# EUROPEAN PATENT SPECIFICATION

(11) **EP 4 262 834 B1**
(45) Date of publication and mention of the grant of the patent: **18.03.2026**
(21) Application number: 21841007.4
(22) Date of filing: 17.12.2021
(51) Int. Cl.: A61K 35/745, A61K 35/747, A61P 25/00, A61P 25/22, A61P 25/24

(54) **COMPOSITIONS COMPRISING LACTOBACILLUS REUTERI PBS072 AND BIFIDOBACTERIUM BREVE BB077 OR BIFIDOBACTERIUM LACTIS BL050 AND THERAPEUTIC USES THEREOF**
ZUSAMMENSETZUNGEN, DIE LACTOBACILLUS REUTERI PBS072 UND BIFIDOBACTERIUM BREVE BB077 ODER BIFIDOBACTERIUM LACTIS BL050 UMFASSEN, UND DEREN THERAPEUTISCHE VERWENDUNG
COMPOSITIONS COMPRENANT LACTOBACILLUS REUTERI PBS072 ET BIFIDOBACTERIUM BREVE BB077 OU BIFIDOBACTERIUM LACTIS BL050 ET UTILISATIONS THÉRAPEUTIQUES DE CELLES-CI

(30) Priority: 17.12.2020 IT 202000031223
(43) Date of publication of application: 25.10.2023
(73) Proprietor: SYNBALANCE SRL, 21040 Origgio (VA) (IT)
(72) Inventor: MALANCHIN, Rosella, 21040 Origgio (VA) (IT); PIANGIOLINO, Cristiana, 21040 Origgio (VA) (IT); CASTEGNARO, Silvia, 21040 Origgio (VA) (IT); CARLOMAGNO, Federica, 21040 Origgio (VA) (IT); PESCIAROLI, Chiara, 21040 Origgio (VA) (IT)
(74) Representative: Bianchetti & Minoja with Trevisan & Cuonzo IPS SRL
(86) International application number: PCT/IB2021/061919
(87) International publication number: WO 2022/130317

(56) References cited:
- EP-A1- 2 251 022
- WO-A1-2012/170021
- WO-A1-2015/170158
- WO-A1-2016/065419
- TW-A- 201 333 194
- TONG JIAQI ET AL: "Nutraceuticals and probiotics in the management of psychiatric and neurological disorders: A focus on microbiota-gut-brain-immune axis", BRAIN, BEHAVIOR AND IMMUNITY, ACADEMIC PRESS, SAN DIEGO, CA, US, vol. 90, 1 September 2020 (2020-09-01), pages 403 - 419, XP086277226, ISSN: 0889-1591, [retrieved on 20200901], DOI: 10.1016/J.BBI.2020.08.027

## Description

### Field of the invention

Probiotic compositions are disclosed herein comprising a strain belonging to the *Lactobacillus reuteri* species in association with a strain belonging to the *Bifidobacterium* genus selected from the species: *Bifidobacterium breve* or *Bifidobacterium lactis,* useful in the prevention and/or treatment of pathological conditions related to stress, selected from the group comprising impairment cognitive function, insomnia, anxiety, baby blues and post-partum depression, menopausal depression, generalized anxiety disorder, major depressive disorder, panic attacks, attention deficit disorder or combinations thereof.

### Background of the invention

Stress is a functional response by which the body reacts to an external stimulus in an attempt to restore inner balance.

Stress symptoms are many and the most frequent ones may be grouped into four categories:
- physical symptoms, such as stomach pain, nausea, diarrhoea, tachycardia;
- behavioural symptoms, such as compulsive eating, more frequent alcohol consumption;
- emotional symptoms: tension, anger, nervousness, anxiety, agitation, sleep disorders;
- cognitive symptoms: difficulty concentrating and making decisions, memory impairment.

According to the triggered event, stress can be acute or chronic.

An acute stressful event such as childbirth can lead to "baby blues" or even to post-partum depression, considered a sub-class of the main depressive disorder (MDD).

As confirmed by the World Health Organization (WHO) and recent publications, post-partum depression is a condition that affects around 10-15% of the world population.

On the other hand, the "baby blues" is a very common condition, affecting about 80% of new mothers. This condition usually occurs a few days after birth and is caused by hormonal changes and lack of sleep; generally, it resolves between the eighth and sixteenth week after the birth of the new-born. New mothers with baby blues show various symptoms such as extreme fatigue, sleep disturbances, extreme guilt or a sense of inadequacy towards the new-born. Furthermore, this condition can lead to reduced interaction and less sense of attachment between mother and child.

Chronic stress may be work related. This condition can affect any employee with repercussions on their quality of life. Work-related stress is considered a real problem worldwide: 80% of American workers feel stressed, half of them ask for help to understand how to manage stress; China reports highest increase in workplace stress; while in Australia the annual average of lost working days by employees is about 3.2, with an impact on the Australian economy for a value of 14.2 billion dollars. In England, around 13.7 million working days are lost each year as a result of work-related stress with an annual cost of around £ 28.3 billion.

More recently, not only work but also study is a source of chronic stress: the culture of competition as well as the uncertainty of the future are sources of stress and depression. This form of stress is perceived by the totality of students: about 20% of American students have experienced symptoms of depression as well as 87% of English students who declare they suffer from stress, 80% declare they feel sad and 77% claims to feel powerless and anxious. In Italy, on the other hand, 800,000 young people between the ages of 12 and 25 say they are not satisfied with their lives.

The study is therefore no longer seen as a moment of personal growth, but only as an interlude to achieve a good result. For young people, therefore, it is not the person who is valued, but rather the mark with which he/she presents him/her-self (enrolment in prestigious universities, first job application), emphasizing most of all, not the curiosity and the importance of learning and increasing their personal background, but the fear of failure which often leads to depression and loss of vitality.

WO2016/065419 discloses oral compositions comprising *Bifidobacterium breve* and *Lactobacillus reuteri* for use in treating or preventing at least one symptom of depression (insomnia, short term memory-loss) anxiety or a depressive or anxiety-related disorder.

TW201333194 discloses a composition comprising *Lactobacillus reuteri* DSM 17938, optionally in combination with *Bifidobacterium lactis* orally administered for promoting healthy establishment of cognitive function and/or preventing or repairing memory loss, cognitive deficits or for reducing the severity of cognitive deficits in young mammals.

WO2012/170021 discloses a nutritional composition comprising a probiotic selected from the group consisting of *Bifidobacterium lactis* CNCM 1-3446, *Lactobacillus reuteri* ATCC55730, *Lactobacillus reuteri* DSM-17938, *Bifidobacterium breve* CNCM 1-3865 and combinations thereof.

Tong Jiaqi et al. (2020); Nutraceuticals and probiotics in the management of psychiatric and neurological disorders: A focus on microbiota-gut-brain-immune axis. Brain, Behavior, and Immunity (90), 403-419, discloses nutraceuticals and probiotics in the management of psychiatric and neurological disorders.

In light of the above, there is still a need to identify alternative compositions effective in the prevention and/or treatment of acute or chronic conditions linked to stress.

### Brief description of figures

Figure 1 shows the quantification of acid γ-aminobutyric acid (GABA) neurotransmitter levels in the culture medium of the neuronal cell line SK-N-DZ after inducing a stressor stimulus with cortisol and in the same system treated with probiotics. CTR -: basal levels of GABA; CTR +: GABA levels following treatment with cortisol causing a harmful response to stress; LR - PBS072: activity of *L. reuteri* PBS072 following a stressor stimulus; BB - BB077: activity of *B. breve* BB077 following a stress stimulus; BL - BL050: activity of *B. lactis* BL050 following a stressful stimulus.

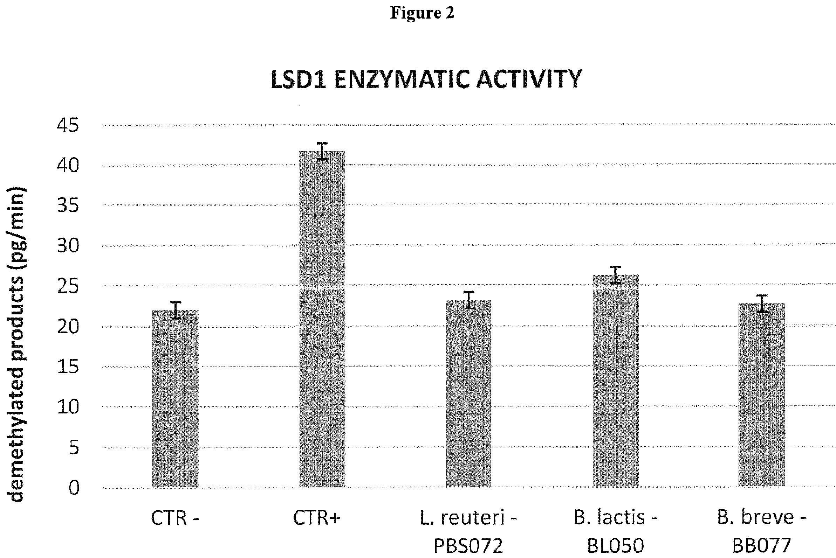

Figure 2 shows the specific activity of the enzyme Demethylase Specific Lysine-1 (LSD1) determined by the quantification of demethylated products in the culture medium of the SK-N-DZ neuroblast cell line following the induction of a stress stimulus and in the same system treated with probiotics. CTR -: basal activity of the LSD1 enzyme; CTR +: enzyme activity following treatment with cortisol causing a harmful response to stress; LR - PBS072: activity of *L. reuteri* PBS072 following a stressful stimulus; BB - BB077: activity of *B. breve* BB077 following a stress stimulus; BL - BL050: activity of *B. lactis* BL050 following a stressful stimulus.
Figure 3 shows the levels of serotonin within the culture medium of intestinal cells Caco2 following stress induced by the addition of cortisol in the medium and in the same system in the presence of probiotics. CTR -: basal serotonin levels, CTR +: serotonin levels following cortisol treatment, causing a harmful enteric stress response; LR - PBS072: activity of *L. reuteri* PBS072 following a stressful stimulus; BB - BB077: activity of *B. breve* BB077 following a stress stimulus; BL - BL050: activity of *B. lactis* BL050 following a stressful stimulus.
Figure 4 shows the increase in the number of correct answers related to a reduction in the number of errors with respect to time 0 - T0 (value expressed as a percentage). (* p <0.05 vs T0; *** p <0.001 vs T0).
Figure 5 shows the increase in the number of correct answers related to a reduction in the response time compared to time 0 - T0 (value expressed as a percentage). (*** p <0.001).
Figure 6 shows the improvement in psychophysiological markers compared to the start of treatment (time 0 - T0) expressed as a percentage. (** p <0.01; * p <0.05 vs T0).
Figure 7 shows a diagram representing the conduct of the clinical study to evaluate the effectiveness of the compositions in subjects subjected to work-related stress.

### Summary of the invention

The present invention is defined in claims 1-15 and relates to compositions comprising a probiotic combination comprising a strain belonging to the *Lactobacillus reuteri* species, that is *Lactobacillus reuteri* PBS072, in association with a strain belonging to the *Bifidobacterium genus* selected from the species: *Bifidobacterium breve* or *Bifidobacterium lactis, i.e. Bifidobacterium breve* BB077 or *Bifidobacterium lactis* BL050.

Furthermore, the invention relates such compositions for use in the prevention and/or treatment of acute or chronic pathological conditions linked to stress selected from the group comprising impairment (deterioration) of cognitive functions, anxiety, insomnia, baby blues or post-partum depression, menopausal depression, generalized anxiety disorder, major depressive disorder, panic attacks, attention deficit disorder or combinations thereof.

### Detailed description of the invention

Compositions are disclosed herein comprising a probiotic combination comprising a strain belonging to the *Lactobacillus reuteri* species (also known as: *Limosilactobacillus reuteri*) in association with a strain belonging to the *Bifidobacterium genus* selected from the species: *Bifidobacterium breve* or alternatively *Bifidobacterium lactis.*

It has surprisingly been found that compositions comprising a probiotic combination comprising a strain belonging to the *Lactobacillus reuteri* species in association with a strain belonging to the *Bifidobacterium breve* or alternatively *Bifidobacterium lactis* species, are effective in the prevention and/or treatment of pathological conditions related to stress such as impairment/deterioration of cognitive functions, in particular due to work-related stress; anxiety, especially during the examination phase; insomnia; baby blues; postpartum depression; depression in menopause; generalized anxiety disorder; major depressive disorder; panic attacks; attention deficit disorder or their combinations.

According to the present invention, "prevention and/or treatment of impairment of cognitive functions" means the prevention and/or treatment of cognitive symptoms such as difficulty concentrating (attention deficit) and difficulty making decisions, reduced short-term memory. The term "impairment" according to the present invention means a reduction with respect to the normal physiological function of short-term memory, attention and the ability to make decisions.

According to the invention, the compositions comprise a combination of a strain for each of the present different probiotic species, preferably as the only active ingredients, in particular the compositions comprise a probiotic combination comprising *Lactobacillus reuteri* PBS072 in association with *Bifidobacterium breve* BB077 or alternatively with *Bifidobacterium lactis* BL050.

The compositions comprise a combination of *Lactobacillus reuteri* PBS072 in association with *Bifidobacterium breve* BB077 or alternatively a combination of *Lactobacillus reuteri* PBS072 in association with *Bifidobacterium lactis* BL050.

The strain of *Lactobacillus reuteri* (*L. reuteri* or LR) called "PBS072" was deposited at DSMZ - DeutscheSammlung von Mikroorganismen und Zellkulturen GmbH under the access number "DSM 25175" on 09/14/2011 under the Budapest Treaty.

The strain of *Bifidobacterium breve (B. breve* or BB) named "BB077" has been deposited at BCCM (Belgian Coordinated Collections of Micro-organisms) - LMG (Laboratorium voor Microbiologie - Bacteriënverzamelig at the access number "LMG P-30157" dated 09/06/2017 pursuant to the Budapest Treaty.

The strain of *Bifidobacterium lactis (B. lactis* or BL) called "BL050" was deposited at DSMZ - DeutscheSammlung von Mikroorganismen und Zellkulturen GmbH at the access number "DSM 25566" on 17/01/2012 pursuant to the Treaty of Budapest.

The present invention relates to compositions comprising, as active ingredients, a probiotic combination comprising the *Lactobacillus reuteri* PBS072 strain in association with *Bifidobacterium breve* BB077 strain or alternatively *Bifidobacterium lactis* BL050 and at least one physiologically acceptable excipient and/or carrier.

The compositions can be formulated by conventional methods. Preferred forms of administration are solid formulations, for example hard capsules, single or double sachets, sticks, oro-soluble sticks, tablets, granules, or liquid formulations, for example vials with single or multi-dose measuring caps, multi-dose dispersions in oily phase, drops, syrups, multi-phase emulsions, etc.

The strain of *Lactobacillus reuteri* PBS072, is present in the composition in percentage by weight on the total weight of the probiotic combination from 20% to 80%, preferably from 30% to 70%, more preferably it is equal to 55%.

The strain of *Lactobacillus reuteri* PBS072, may be present in each single unit dose in an amount ranging from 0.5 to 5 billion CFU, preferably in an amount ranging from 1 to 4 billion CFU, more preferably it is present in amount equal to 2 billion CFU.

The strain *Bifidobacterium breve* BB077, is present in the composition in percentage by weight on the total weight of the probiotic combination from 10% to 60%, preferably from 20% to 50%, more preferably it is equal to 40%.

The strain *Bifidobacterium breve* BB077, may be present in each single unit dose in an amount ranging from 0.5 to 5 billion CFU, preferably in an amount ranging from 1 to 4 billion CFU, more preferably it is present in an amount equal to 2 billion CFU.

The strain *Bifidobacterium lactis* BL050, is present in the composition in percentage by weight on the total weight of the probiotic combination from 10% to 60%, preferably from 20% to 50%, more preferably it is equal to 40%.

The strain *Bifidobacterium lactis* BL050, may be present in each single unit dose in an amount ranging from 0.5 to 5 billion CFU, preferably in an amount ranging from 1 to 2 billion CFU, more preferably it is present in amount equal to 2 billion CFU.

The two species *Lactobacillus reuteri* PBS072 and *Bifidobacterium breve* BB077 may be present in a weight ratio of 1:0.7 or in a ratio of 1:1 if expressed in CFU. Alternatively, the two species *Lactobacillus reuteri* PBS072 and *Bifidobacterium lactis* BL050 may be present in a weight ratio equal to 1:0.7 or in a 1:1 ratio if expressed in CFU.

The probiotic compositions of the invention can be administered orally.

A further object of the present invention is the compositions comprising a probiotic combination comprising the *Lactobacillus reuteri* PBS072 strain in association with *Bifidobacterium breve* BB077 or alternatively *Bifidobacterium lactis* BL050, as a medicament, in particular for use in the prevention and/or treatment of acute or chronic pathological conditions linked to stress selected from impairment/deterioration of cognitive function, anxiety, baby blues, post-partum depression, insomnia, menopausal depression, generalized anxiety disorder, major depressive disorder, panic attacks, attention deficit disorder or combinations thereof. Preferably acute or chronic conditions related to stress are selected from impairment of cognitive functions, anxiety, baby blues, post-partum depression, insomnia, or combinations thereof.

The impairment of cognitive functions due to stress, is caused by high pressure situations such as facing exams, a particular working moment (work-related stress) or period as the post-partum.

According to an aspect of the invention, the compositions of the invention are useful to improve (prevent and/or treat the reduction) cognitive functions, or to restore short-term memory, attention and/or decision-making ability when altered by stress, particularly from work or study related stress or from childbirth stress.

According to a further embodiment of the invention, the compositions of the invention are useful in the prevention and/or treatment of postpartum depression and the condition known as "baby blues".
The following examples further illustrate the invention.

### Examples

### Formulative examples

### Example 1

A stick composition has been prepared, containing:

| **INGREDIENTS** | **% p/p** | **BILLIONS CFU/DOSE** |
|---|---|---|
| *Lactobacillus reuteri* PBS072 | 4.66 | 2 |
| *Bifidobacterium breve* BB077 | 3.11 | 2 |
| FOS 93% | 3.60 | - |
| Inulin 90% | 3.703 | - |
| Folic Acid | 0.003 | - |
| Vitamin B12 | 0.035 | - |
| Vitamin B6 | 0.022 | - |
| Sorbitol | 9.00 | - |
| Sucralose | 0.08 | - |
| Flavour | 1.30 | - |
| Silicon dioxide | 1.00 | - |
| Maltodextrin | 73.487 | - |

### Example 2

A composition in capsule form has been prepared, containing:

| **INGREDIENTS** | **% p/p** | **BILLIONS CFU/DOSE** |
|---|---|---|
| *Lactobacillus reuteri* PBS072 | 9.71 | 2 |
| *Bifidobacterium breve* BB077 | 6.47 | 2 |
| Maltodextrin | 65.37 | - |
| Capsule DR size 0 | 18.45 | - |

### Example 3

A composition in capsule form has been prepared, containing:

| **INGREDIENTS** | **% p/p** | **BILLIONS CFU/DOSE** |
|---|---|---|
| *Lactobacillus reuteri* PBS072 | 9.71 | 2 |
| *Bifidobacterium breve* BB077 | 6.47 | 2 |
| Vitamin mix: Vitamins B1, B2, B6, B12, Folic acid, Vitamin D, Niacin, Pantothenic acid, Vitamin E | 8.16 | - |
| Maltodextrin | 57.21 | - |
| Capsule DR size 0 | 18.45 | - |

### Example 4

A composition in double stick form has been prepared, containing:

| **INGREDIENTS** | **% p/p** | **BILLIONS CFU/DOSE** |
|---|---|---|
| *Lactobacillus reuteri* PBS072 | 2.33 | 2 |
| *Bifidobacterium lactis* BL050 | 1.55 | 2 |
| Withania somnifera | 8.33 | - |
| Maltodextrin | 50.79 | - |
| Corn starch | 10.00 | - |
| Silicon dioxide | 2.00 | - |
| Sorbitol | 16.67 | - |
| Flavour | 8.33 | - |

### Example 5

A oro-soluble stick composition has been prepared, containing:

| **INGREDIENTS** | **% p/p** | **BILLIONS CFU/DOSE** |
|---|---|---|
| *Lactobacillus reuteri* PBS072 | 1.75 | 2 |
| *Bifidobacterium lactis* BL050 | 1.17 | 2 |
| Acacia fibre | 50.00 | - |
| Maltodextrin | 22.58 | - |
| Corn starch | 7.50 | - |
| Silicon dioxide | 1.00 | - |
| Sorbitol | 15.00 | - |
| Flavour | 1.00 | - |

### Experimental examples

The following experimental examples relate to the evaluation of the efficacy of the compositions of the invention in the prevention and/or treatment of the conditions described above.

### Examples 6 - Cognitive Functions

A clinical study was conducted to determine the effectiveness of the composition reported in the Example 1 in preventing and / or treating the impairment of cognitive functions. This study is based on the *in-vitro* results obtained from the composition of Example 1, further verified in a pilot clinical study on humans.

### I. In-vitro results

### Materials and methods

For the evaluation of serotonin levels, the intestinal cell line Caco2 was selected, mimicking the intestinal epithelium considering that most of the serotonin is produced in the intestine. Instead, for the evaluation of GABA levels and the enzymatic activity of the LSD1 enzyme, the neuroblast cell line SK-N-DZ (ATCC CRL-2149) was selected.

### Clinical study End-points:

To determine the effectiveness of the study, various experimental models were studied as follows:
- cell line in the presence of the single probiotic strains of the invention (LR - PBS072: *L. reuteri* PBS072; BB - BB077: *B. breve* BB077; BL - BL050: *B. lactis* BL050) and in the presence of cortisol used to induce a stressful situation;
- cell line treated with cortisol in the absence of probiotics (CTR +);
- untreated cell line (CTR-).

At the end of the 48 hours of incubation the following biochemical measurements were carried out:
❖ quantification of the expression of γ-aminobutyric acid (GABA), an inhibitory neurotransmitter involved in sleep regulation (cell line: SK-N-DZ);
❖ evaluation of the specific demethylase activity of the LSD1 enzyme, an enzyme that functions at the epigenetic level, involved in the mechanisms of neuronal plasticity and adaptation (cell line: SK-N-DZ);
❖ quantification of the expression of serotonin, a precursor in the biosynthesis of melatonin, involved in the regulation of the sleep/wake cycle (Caco2 cell line).

### Results

### GABA levels evaluations

The synthesis of γ-aminobutyric acid was greatly influenced by the action of probiotics. GABA levels significantly decreased in the neuronal line treated exclusively with cortisol (CTR +).

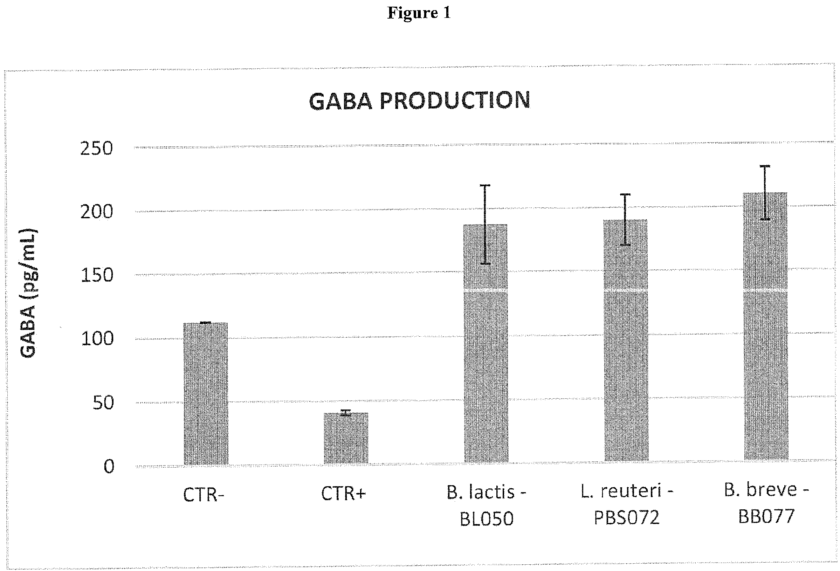

The three probiotic strains, *L. reuteri* PBS072, *B. breve* BB077 and *B. lactis* BL050 have been shown not only to reverse the effect caused by the presence of cortisol, but to increase γ-aminobutyric acid levels above the GABA baseline (CTR-) in the system. This is to be considered a positive result regarding sleep modulation, influence on the mood and maintain high concentration in high stress situations. In fact, GABA is an inhibitory neurotransmitter able to down-regulate the excitatory signals (involved in the mechanisms of awakening) and those arising as a result of a reaction following social stress. The results are reported in Figure 1 and Table 1.

**Table 1**

| **GABA production in the SK-N-DZ neuronal line** | | | |
|---|---|---|---|
| | Average | Standard Deviation | % Var vs CTR- |
| CTR- | 112.1 | 0.5 | |
| CTR+ (with Cortisol) | 40.6 | 2 | |
| BL - BL050 | 187.2 | 31 | 67% |
| LR - PBS072 | 190.2 | 20 | 70% |
| BB -BB077 | 210.4 | 20.9 | 88% |

### Evaluation of the enzymatic activity of the LSD1 enzyme

The probiotic strains of the invention have been shown to have a positive effect on the modulation of the enzymatic activity of the specific lysine demethylase enzyme (LSD1), a histone H3 *"eraser"* that regulates the transcription of genes involved in neuronal plasticity. High enzymatic activity is associated with an increase in the response to social stress. The restoration of the enzymatic activity of the LSD1 is associated with a correct transcription activity of the genes involved in the modulation of the stress response.

In this experimental model, the enzymatic activity in the CTR+ is increased due to the presence of cortisol in the system.

The data reported in table 2 show how the presence of the probiotic strains *L. reuteri* PBS072, *B. breve* BB077 and *B. lactis* BL050 in the system is able to restore normal activity of LSD1 enzyme in the presence of cortisol.

**Table 2**

| LSD1 enzymatic activity in the SK-N-DZ neuronal line | | | |
|---|---|---|---|
| | Average | Standard Deviation | % Var vs CTR+ |
| CTR - | 22 | 1.7 | |
| CTR+ (with Cortisol) | 41.7 | 3.1 | |
| LR - PBS072 | 23.2 | 1.8 | -44% |
| BL - BL050 | 26.2 | 0.5 | -37% |
| BB - BB077 | 22.7 | 1.8 | -46% |

### Serotonin levels evaluation

In this experimental model, the ability of the probiotic strains to restore serotonin levels in an intestinal cell line following the addition of cortisol to the system was evaluated.

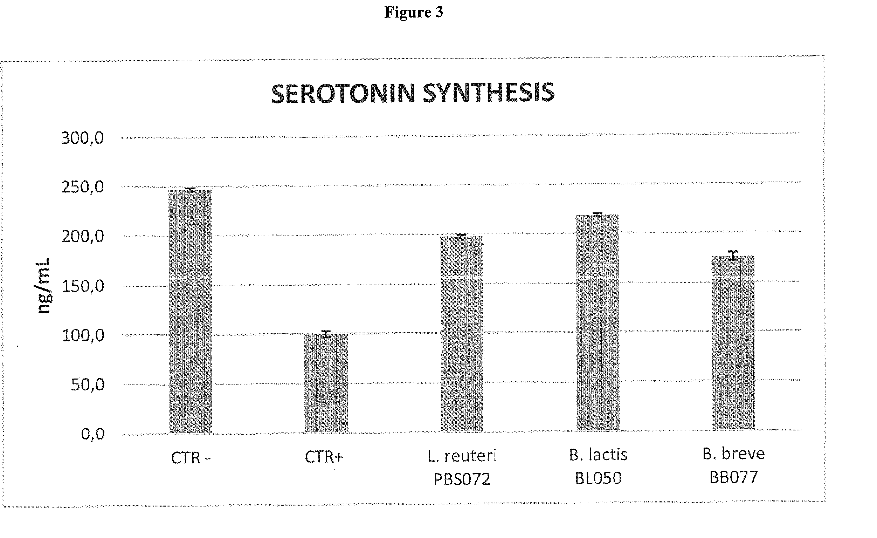

This cell line was selected because about 90% of serotonin is produced at the enteric level. The presence of cortisol in the system induced a notable reduction of serotonin (CTR +); on the contrary, the presence of the probiotic strains *L. reuteri* PBS072, *B. breve* BB077 and *B. lactis* BL050 in the system counteracted the negative activity of cortisol, bringing back serotonin to values comparable to the CTR-. The results are reported in Figure 3 and Table 3.

**Table 3**

| Serotonin expression in the Caco2 enteric line | | | |
|---|---|---|---|
| | Average | Standard Deviation | % Var vs CTR- |
| CTR - | 246.9 | 1.7 | |
| CTR+ (with Cortisol) | 99.2 | 3.1 | -59.8% |
| LR - PBS072 | 197.5 | 1.8 | -20.0% |
| BL - BL050 | 218.3 | 1.8 | -11.6% |
| BB - BB077 | 175.6 | 4.4 | -28.9% |

### II. Clinical Evaluation

### Materials and methods

The clinical evaluation conducted was an open study to evaluate the efficacy of the product orally administered. The probiotic composition reported in the Example 1, containing the combination of *Lactobacillus reuteri* PBS072 and *Bifidobacterium breve* BB077, was administered in order to observe the effect on cognitive functions (problem solving, attention, short-term memory) and in general on stress perceived (salivary cortisol levels, skin conductance, sleep quality and anxiety) in a cohort of 30 students aged 18-30 over a 28-day period.

The study reported significant results, compared to the starting condition, as regards both cognitive functions and psycho-physiological markers.

The study was divided into two experimental stages:
- T0 = in which the candidates were selected and subjected to specific psychological tests to evaluate the different cognitive functions. Salivary samples were also taken to measure cortisol levels and skin conductance levels. The assessment of sleep quality and anxiety were carried out by completing specific questionnaires.
- T28 = at the end of the study, the test of T0 were carried out again.

The effectiveness of the treatment was assessed on the basis of the results obtained from the following tests, questionnaires and biochemical analyses:
❖ Attention;
❖ Short Term Memory;
❖ Executive Performance;
❖ Self-assessment questionnaire on state and trait anxiety (STAI);
❖ Self-assessment questionnaire on sleep quality (Athens insomnia scale);
❖ Skin Conductance;
❖ Salivary cortisol.

### Results

At the beginning, 30 students were selected because they were subjected to severe stress during the examination period, 3 volunteers did not complete the study due to medical needs. Thus, 27 students completed the study, demographic characteristics are shown in table 4.

**Table 4**

| | |
|---|---|
| | |

| Gender | N. |
|---|---|
| Male | 8 |
| Female | 21 |
| Age (±SEM) | 23.4±4.6 |

Generally, a significant improvement was observed at the end of treatment with the probiotic composition reported in Example 1 (28 days) compared to the beginning of treatment.

### Cognitive functions improvement

### Short-term memory

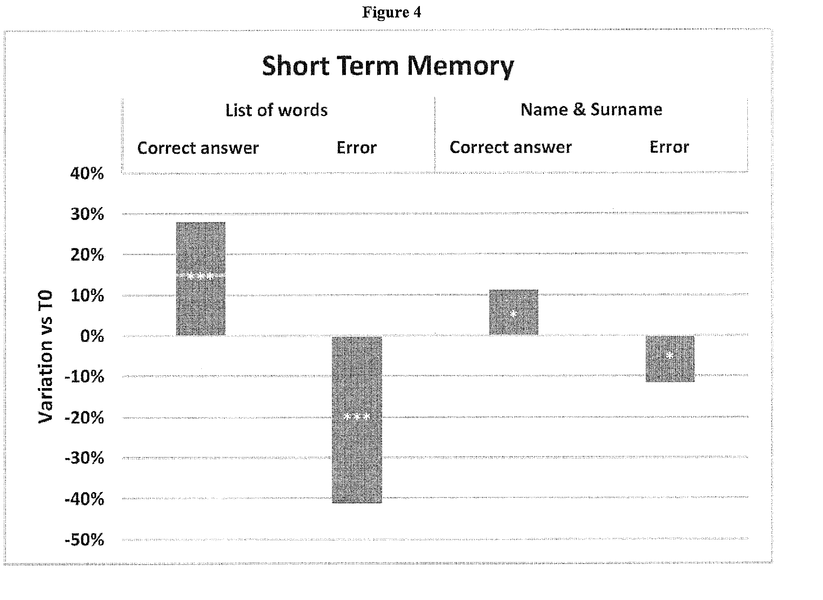

Subjects performed two tests to assess short-term memory improvement. These tests are based on the subject's ability to memorize the largest number of words in a list (in one case random words and in the other an association of names and surnames). At the end of the time allowed for memorization, the subjects were subjected to a test designed to act as "interference" as the volunteers were asked to shift their concentration to something else. At the end of the interference test, subjects had to write as many words as they remembered. At the end of the treatment, the results showed that the administration of the probiotic composition reported in Example 1 increased the short-term memory of the subjects with a significant improvement in the number of correct answers and a reduction in the number of errors with respect to the beginning. The results, represented as a percentage variation compared to the beginning of the treatment, are shown in Figure 4 and Table 5.

**Table 5**

| **Short-term memory** | | | | |
|---|---|---|---|---|
| | **List of words** | | **Name & Surname** | |
| | **% Var vs T0** | | **% Var vs T0** | |
| | **Correct Answer** | **Errors** | **Correct Answer** | **Errors** |
| Active product | 27.95 *** | -41.06*** | 11.17* | 11.56* |

| | | | | |
|---|---|---|---|---|
| * p<0.05 vs T0; *** p<0.001 vs T0 | | | | |

### Attention and executive function improvement

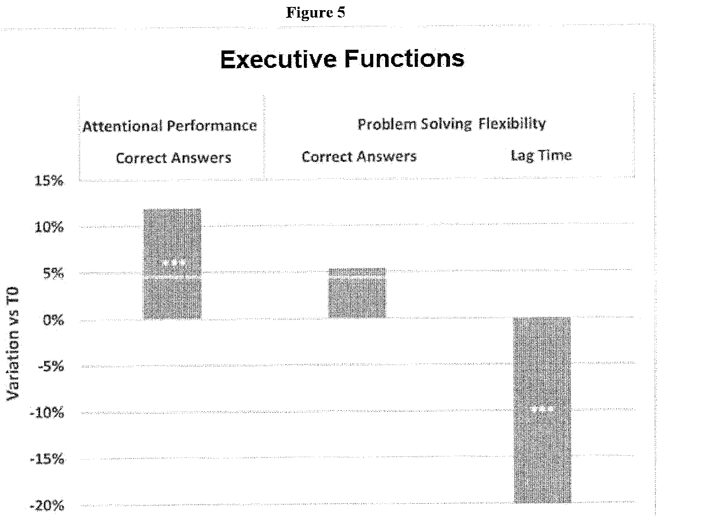

Attention was assessed through the "Divided Attentional Performance Test" in order to assess the ability of the subjects to maintain high levels of concentration in the presence of two irregular stimuli. The subjects were asked to answer questions in presence of both acoustic and visual interference. Instead, to evaluate problem solving skills, the subjects completed the "Wisconsin card sorting test", which aims to evaluate their ability to respond to a sequence of inputs, providing the correct feedback in a short period of time. From the analysis of the "Divided Attentional Performance Test" and the "Wisconsin card sorting test" it was possible to observe that subjects showed an improvement both in the percentage of the number of correct answers and in the responsiveness, compared to the beginning of the treatment, demonstrating a prompt response in taking decisions in a short time. The results are shown in Figure 5 and Table 6 as a percentage change from the start of treatment and are statistically significant.

**Table 6**

| | **Problem Solving Flexibility** | | **Divided Attention** | |
|---|---|---|---|---|
| | **% Var vs T0** | | **% Var vs T0** | |
| | **Correct Answers** | **Errors** | **Correct Answers** | **Errors** |
| Active Product | 5.36 | -20.52*** | 11.91*** | -2.04 |

| | | | | |
|---|---|---|---|---|
| *** p<0.001 vs T0 | | | | |

### Psycho-physiological markers

To get a complete picture of subjects' quality of life improvement, several analysis were assessed both at the physiological (measurement of salivary cortisol, measurement of skin conductance) and psychological level (questionnaires on sleep quality and anxiety).

### Skin conductance

The skin conductance was measured during the experimental sessions, in order to observe physiological changes linked to the activation of the sympathetic/parasympathetic system as a marker of anxiety-induced stress.

### Salivary Cortisol

Salivary samples were processed to measure salivary cortisol levels: an increased concentration is physiologically linked to a growth of stress sensation and sleep problems.

### Sleep Quality

Sleep quality was assessed using the eight-question "Athens insomnia scale" questionnaire with an answer scale from 1 to 4. Positive results were assessed on the basis of the sum of the individual scores associated with the 8 questions.

### Anxiety

Anxiety was assessed through the "State-Trait Anxiety Inventory" (STAI) questionnaire, in order to evaluate both anxiety as an intrinsic characteristic and as a state caused by a specific event.

Overall, the results show that the assumption of the probiotic composition of Example 1 has brought about a significant improvement in the psycho-physiological markers involved in stress. In fact, a significant reduction in salivary cortisol and skin conductance was observed; at the same time an improvement in both the quality of sleep and anxiety was observed.

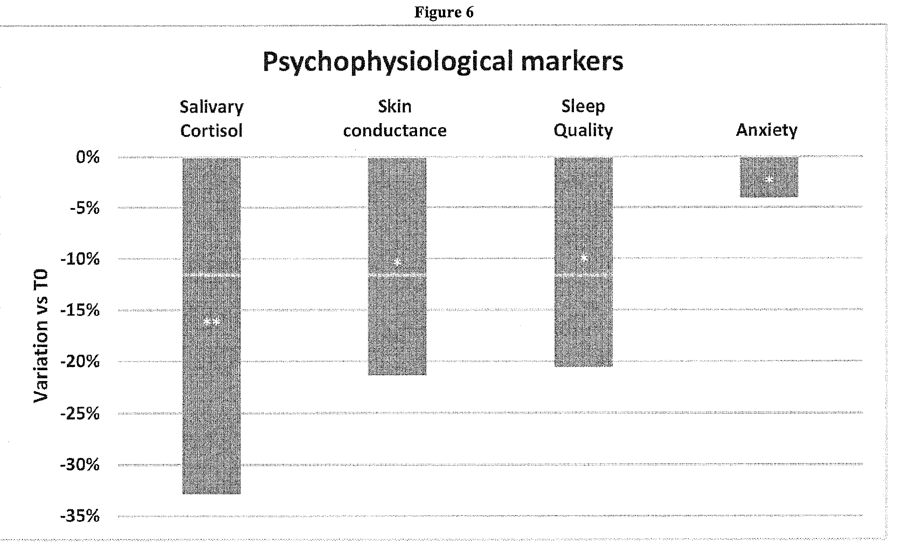

The results are shown in Figure 6 and Table 7 as a percentage variation with respect to the beginning of the treatment.

**Table 7**

| | **% Var vs T0** | | | |
|---|---|---|---|---|
| | **Salivary Cortisol** | **Skin Conductance** | **Sleep Quality** | **Anxiety** |
| Active product | -33.00** | -21.39* | -21.00 * | -4.08* |

| | | | | |
|---|---|---|---|---|
| * p<0.05 vs T0; ** p<0.01 vs T0 | | | | |

### Example 7 - Work related stress

A clinical study was conducted in a randomized cross-over mode to evaluate the efficacy of the probiotic composition reported in Example 2 containing *Lactobacillus reuteri* PBS072 and *Bifidobacterium breve* BB077 in the prevention and/or treatment of work-related stress symptoms in employees. Volunteers took one capsule of the product per day, Active Treatment A or Placebo Treatment B, for 30 days (Treatment A or Treatment B), followed by a 4-week break (wash-out period). At the end of the wash-out period, each volunteer took again one capsule per day for 30 days of the product (Treatment A or Treatment B). The two treatments were alternate: Treatment A (composition containing probiotics reported in Example 2) and Treatment B placebo (probiotic-free) shown in table 8.

**Table 8**

| **INGREDIENTS** | **% p/p** |
|---|---|
| Maltodextrin | 81.55% |
| Capsule size DR | 18.45% |

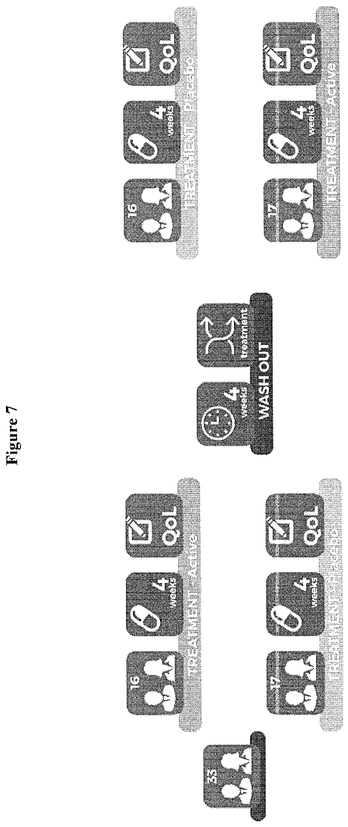

The assignment of each treatment was randomized, the volunteers were not aware of which product they were taking during the two study periods. Figure 7 shows a diagram representing the development of the study. At the beginning and at the end of each treatment, meetings were organized with the managers of the study to determine the emotional state of the volunteers. In the meetings, volunteers were asked to fill out questionnaires to assess their state of stress along with the quality of sleep in the last month.

### Materials and methods

To evaluate the effectiveness of both treatments in the two parallel groups, at the beginning and at the end of each treatment, the volunteers were given questionnaires to assess both stress and sleep quality.

The subjects were randomly administered with the probiotic-based composition shown in Example 2 containing *Lactobacillus reuteri* PBS072 and *Bifidobacterium breve* BB077 or the placebo product shown in table 8.

The study was divided into four time points:
- T0 = candidates, once selected, signed the informed consent and filled out the different questionnaires for the evaluation of their basal emotional state and the quality of sleep. At the end of this, the product of Treatment A or Treatment B was delivered.
- T30 = At the end of the first treatment, the candidates filled out the questionnaires to evaluate the effect of the first treatment.
- T60 = Beginning of the second treatment (A or B). Candidates completed questionnaires to assess their condition at the beginning of the second treatment.
- T90 = End of the second treatment. Candidates completed the questionnaires one last time to evaluate the effect of the second treatment.

The effectiveness of the treatment was assessed on the basis of the results obtained from the following questionnaires:
- *Profile of Mood States Questionnaire* (POMS): used to assess psychological distress;
- *Pittsburgh Sleep Quality Index* (PSQI) to assess sleep quality in the last month.

### Results

At the beginning, 33 employees were selected because they were subjected to high stress in the workplace, but 9 volunteers did not complete the study due to medical needs. 24 employees completed the study, demographic characteristics are shown in table 9.

**Table 9**

| Gender | |
|---|---|
| Male | 10 |
| Female | 23 |
| Average Age | 35.4 |

### Mood improvement

The mood of each subject was assessed through the validated "Profile of Mood States" (POMS) questionnaire in order to observe an improvement in the overall mood; this improvement can be observed through a reduction in the overall score obtained.

The group treated with the probiotic composition shown in Example 2 reported statistical significant results, demonstrating an improvement in the general mood state. Table 10 shows the results in the form of a percentage variation with respect to T0 (beginning of treatment).

**Table 10**

| | **Variation % vs T0** |
|---|---|
| | Mood improvement |
| Treatment A - Active product | -43.08*# |
| Treatment B - Placebo | 27.4 |

| | |
|---|---|
| * p<0.05 vs T0; # p=0.057 vs Placebo | |

### Sleep quality evaluation

The sleep quality of each subject was assessed through the validated "Pittsburgh Sleep Quality Index" (PSQI) questionnaire in order to observe an improvement, that can be detected through a reduction in the overall score obtained.

The group treated with the probiotic composition reported in Example 2 reported statistical significant results, demonstrating an amelioration of the sleep quality. Table 11 shows the results as a percentage variation with respect to T0 (beginning of treatment).

**Table 11**

| | **Variation % vs T0** |
|---|---|
| | Sleep Quality |
| Treatment A - Active Product | -19.8**## |
| Treatment B - Placebo | 20.2 |

| | |
|---|---|
| ** p<0.01 vs T0; ## p<0.01 vs Placebo | |

### Example 8 - Anxiety and post-partum depression

A double-blind, randomized, multicenter clinical study was conducted to evaluate the efficacy of the probiotic composition of Example 3 administered orally in the prevention and/or treatment of postpartum depression and anxiety. The primary objective of this study was to maintain the physiological state of psychophysical well-being of new mothers and reduce the onset possibility of anxiety or stress after childbirth, common situations in both baby blues and post-partum depression.

As secondary targets, the quality of breastfeeding was assessed, identified as the lactation confidence of the new mother and a reduction in the baby's crying (due to colic).

### Materials and methods

To evaluate the effectiveness of the probiotic composition reported in Example 3 containing *Lactobacillus reuteri* PBS072 and *Bifidobacterium breve* BB077, 200 healthy women at the end of their pregnancy were recruited and subsequently randomized in:
▪ 100 volunteers who were asked to take the product containing the probiotic composition of Example 3;
▪ 100 volunteers who were asked to take a vitamin product listed in Table 12.

**Table 12**

| **INGREDIENTS** | **% p/p** |
|---|---|
| Maltodextrin | 56% |
| Vitamin mix: Vitamins B1, B2, B6, B12, Folic Acid, Vitamin D, Niacin, Pantothenic Acid, Vitamin E | 13.6% |
| Magnesium stearate | 3.8% |
| Silicon dioxide | 0.5% |
| Vegetable Capsule DR | 26.1% |

The volunteers were recruited during their last gynaecological visit before giving birth (T-1). The beginning of product intake (T0) takes place within a maximum of 3 (± 1) days after delivery, for a total duration of 90 days (3 months). Visits were scheduled after 45 days (half treatment T45) and at the end of the same, after 90 days (T90). Table 13 shows the activities carried out for each visit.

**Table 13**

| **Phase of the Study** | **Recruitment (T-1)** | **Beginning of the study - Product intake (T0)** | **Intermediate checkpoint (T45)** | **Last visit (T90)** |
|---|---|---|---|---|
| Signature of informed consent | X | - | - | - |
| Subjects enrolment | X | - | X | X |
| Clinical evaluation - Safety of use | - | - | X | X |
| Questionnaire complement with gynaecologist support | - | - | X | X |
| Product distribution | X | - | - | - |
| Return the unused product | - | - | - | X |

The evaluation of the parameters was assessed by specific questionnaire able to determine the improvement of new-mothers and newborns health.

The questionnaires were the following:
- *Edinburgh Postnatal Depression Scale* (EPDS) to evaluate symptoms linked with depression;
- *Breastfeeding Self Efficacy Scale Short Form* (BSES-SF) self-assessment of the quality of breastfeeding, in association with the daily recording of crying episodes.

### Results

The results obtained show how the volunteers treated with the probiotic composition reported in Example 3 reported both an improvement in their emotional state and an amelioration of breastfeeding quality considered as a higher confidence of the mother during lactation and a reduction of crying episodes in the new-born with respect to the control group.

### Mood Evaluation

The mood of new mothers was assessed through the validated Edinburgh Postnatal Depression Scale (EPDS) questionnaire in order to observe an improvement, that can be check through a reduction in the overall score.

The group treated with the probiotic composition shown in Example 3 achieved statistical significant results (p <0.0001), demonstrating an amelioration of new mothers mood. Table 14 shows the results in the two observation times (T45 and T90).

**Table 14**

| **New-mothers mood** | | |
|---|---|---|
| | T45 | T90 |
| Active group | 8.9*** | 6.9*** |
| Control group | 12 | 10.7 |

| | | |
|---|---|---|
| ** p<0.001 vs control | | |

### Breastfeeding quality

The quality of breastfeeding was assessed through the use of the Breastfeeding Self Efficacy Scale Short Form (BSES-SF) questionnaire in order to assess a greater confidence during breastfeeding of the newborn over the study period. This improvement can be observed through an increase in the overall score.

The group that took the probiotic composition shown in Example 3 reported a statistically significant results compared to the control group. Table 15 shows the results relating to the two observation times (T45 and T90).

**Table 15**

| **Breastfeeding Quality** | | |
|---|---|---|
| | T45 | T90 |
| Active group | 51.47*** | 56.31*** |
| Control group | 42.88 | 45.44 |

| | | |
|---|---|---|
| *** p<0.001 vs control | | |

### Crying Episodes

During the treatment period, new mothers considered the number of crying episodes occurred during the treatment. The results show that new-mothers belonging to the active group observed a greater reduction in crying episodes in the new-born with respect to the control. Table 16 shows the results expressed as the percentage of new-mothers who reported a significant reduction (p <0.0001) in crying episodes in the infant in the two observation times (T45 and T90) compared to the control.

**Table 16**

| **Percentage of Volunteers with positive answer** | | |
|---|---|---|
| | T45 | T90 |
| Active group | 81% | 78% |
| Control group | 42% | 43% |

### Conclusions

The strains described in this invention have been shown to modulate stress markers (GABA, Serotonin and LSD1) *in-vitro.* The probiotic compositions of the invention, in particular the compositions reported in Examples 1, 2 and 3, have been shown to exert a positive effect in enrolled subjects (stressed students, employees and post-pregnancy women). The compositions significantly improved the parameters observed in the three studies (cognitive functions, mood, sleep quality, anxiety, etc.). In addition, a significant improvement in the quality of life was observed compared to the beginning of the treatment, both from a physiological and psychological point of view.

## Claims

1. Composition comprising a probiotic combination comprising the following strains:
- *Lactobacillus reuteri* PB S072, and
- *Bifidobacterium breve* BB077 or *Bifidobacterium lactis* BL050;
wherein
the *Lactobacillus reuteri* strain is present in the composition in percentages by weight on the total weight of the probiotic combination from 20% to 80%;
the *Bifidobacterium breve* strain is present in the composition in percentage by weight on the total weight of the probiotic combination from 10% to 60%;
the *Bifidobacterium lactis* strain is present in the composition in percentage by weight on the total weight of the probiotic combination from 10% to 60%.

2. Composition according to claim 1, wherein the *Lactobacillus reuteri* PBS072 strain is present in the composition in percentages by weight on the total weight of the probiotic combination from 30% to 70%, preferably it is equal to 55%.

3. Composition according to claim 1, wherein the *Lactobacillus reuteri* PBS072 strain is present in each single unit dose in a quantity ranging from 0.5 to 5 billion CFU, preferably in a quantity ranging from 1 to 4 billion CFU, more preferably it is present in a quantity equal to 2 billion CFU.

4. Composition according to claim 1, wherein the *Bifidobacterium breve* BB077 strain is present in the composition in percentage by weight on the total weight of the probiotic combination from 20% to 50%, preferably it is equal to 40%.

5. Composition according to claim 1, wherein the *Bifidobacterium breve* BB077 strain is present in each single unit dose in a quantity ranging from 0.5 to 5 billion CFU, preferably in a quantity ranging from 1 to 4 billion CFU, more preferably it is present in an amount equal to 2 billion CFU.

6. Composition according to claim 1, wherein the *Bifidobacterium lactis* BL050 strain is present in the composition in percentage by weight on the total weight of the probiotic combination from 20% to 50%, preferably it is equal to 40%.

7. Composition according to claim 1, wherein the *Bifidobacterium lactis* BL050 strain is present in each single unit dose in a quantity ranging from 0.5 to 5 billion CFU, preferably in a quantity ranging from 1 to 2 billion CFU, more preferably it is present in an amount equal to 2 billion CFU.

8. Composition according to claims 1-5, wherein *Lactobacillus reuteri* PBS072: *Bifidobacterium* brevis BB077 are present in a weight ratio equal to 1:0.7 or in an 1:1 ratio if expressed in CFU.

9. Composition according to claims 1-3, 6 and 7, wherein *Lactobacillus reuteri* PBS072*:Bifidobacterium lactis* BL050 are present in a weight ratio of 1:0.7 or in a 1:1 ratio if expressed in CFU.

10. Composition according to claims 1-9, in oral form.

11. Composition according to claims 1-10, for use as a medicament.

12. Composition according to claims 1-10, for use in the prevention and/or treatment of acute or chronic pathological conditions selected from the group comprising impairment of cognitive functions; insomnia; anxiety; baby blues or postpartum depression; menopausal depression; generalized anxiety disorder; major depressive disorder; panic attacks; attention deficit disorder; or combinations thereof.

13. Composition for use according to claim 12, in the prevention and/or treatment of impairment of cognitive functions; insomnia; anxiety; baby blues or postpartum depression; and their combinations.

14. Composition for use according to claim 13, wherein the impairment of cognitive functions is due to work-related stress.

15. Composition for use according to claim 14, wherein impaired cognitive functions are short-term memory, attention and/or decision-making ability.

## Patentansprüche

1. Zusammensetzung, umfassend eine probiotische Kombination, umfassend die folgenden Stämme:
- *Lactobacillus reuteri* PBS072 und
- *Bifidobacterium breve* BB077 oder *Bifidobacterium lactis* BL050;
wobei
der *Lactobacillus-reuteri-Stamm* in der Zusammensetzung in Gewichtsprozent, bezogen auf das Gesamtgewicht der probiotischen Kombination, in einer Menge von 20 % bis 80 % vorhanden ist;
der *Bifidobacterium-breve-*Stamm in der Zusammensetzung in Gewichtsprozent, bezogen auf das Gesamtgewicht der probiotischen Kombination, in einer Menge von 10 % bis 60 % vorhanden ist;
der *Bifidobacterium-lactis-*Stamm in der Zusammensetzung in Gewichtsprozent, bezogen auf das Gesamtgewicht der probiotischen Kombination, in einer Menge von 10 % bis 60 % vorhanden ist.

2. Zusammensetzung nach Anspruch 1, wobei der Stamm *Lactobacillus reuteri* PBS072 in der Zusammensetzung in Gewichtsprozent, bezogen auf das Gesamtgewicht der probiotischen Kombination, in einer Menge von 30 % bis 70 %, vorzugsweise 55 %, vorhanden ist.

3. Zusammensetzung nach Anspruch 1, wobei der Stamm *Lactobacillus reuteri* PBS072 in jeder Einzeldosis in einer Menge von 0,5 bis 5 Milliarden CFU, bevorzugt in einer Menge von 1 bis 4 Milliarden CFU, stärker bevorzugt in einer Menge von 2 Milliarden CFU, vorhanden ist.

4. Zusammensetzung nach Anspruch 1, wobei der Stamm *Bifidobacterium breve* BB077 in der Zusammensetzung in Gewichtsprozent, bezogen auf das Gesamtgewicht der probiotischen Kombination, in einer Menge von 20 % bis 50 %, vorzugsweise in einer Menge von 40 %, vorhanden ist.

5. Zusammensetzung nach Anspruch 1, wobei der Stamm *Bifidobacterium breve* BB077 in jeder Einzeldosis in einer Menge von 0,5 bis 5 Milliarden CFU, bevorzugt in einer Menge von 1 bis 4 Milliarden CFU, stärker bevorzugt in einer Menge von 2 Milliarden CFU, vorhanden ist.

6. Zusammensetzung nach Anspruch 1, wobei der Stamm *Bifidobacterium lactis* BL050 in der Zusammensetzung in Gewichtsprozent, bezogen auf das Gesamtgewicht der probiotischen Kombination, in einer Menge von 20 % bis 50 %, vorzugsweise in einer Menge von 40 %, vorhanden ist.

7. Zusammensetzung nach Anspruch 1, wobei der Stamm *Bifidobacterium lactis* BL050 in jeder Einzeldosis in einer Menge von 0,5 bis 5 Milliarden CFU, bevorzugt in einer Menge von 1 bis 2 Milliarden CFU, stärker bevorzugt in einer Menge von 2 Milliarden CFU, vorhanden ist.

8. Zusammensetzung nach den Ansprüchen 1 bis 5, wobei *Lactobacillus reuteri* PBS072: *Bifidobacterium brevis* BB077 in einem Gewichtsverhältnis von 1:0,7 oder in einem Verhältnis von 1:1, ausgedrückt in KBE, vorliegen.

9. Zusammensetzung nach den Ansprüchen 1 bis 3, 6 und 7, wobei *Lactobacillus reuteri* PBS072*:Bifidobacterium lactis* BL050 in einem Gewichtsverhältnis von 1:0,7 oder in einem Verhältnis von 1:1, ausgedrückt in KBE, vorliegen.

10. Zusammensetzung nach den Ansprüchen 1 bis 9 in oraler Form.

11. Zusammensetzung nach den Ansprüchen 1 bis 10 zur Verwendung als Arzneimittel.

12. Zusammensetzung nach den Ansprüchen 1 bis 10 zur Verwendung bei der Vorbeugung und/oder Behandlung von akuten oder chronischen pathologischen Zuständen, ausgewählt aus der Gruppe, bestehend aus Beeinträchtigung der kognitiven Funktionen, Schlaflosigkeit, Angstzuständen, Babyblues oder postpartaler Depression, menopausaler Depression, generalisierter Angststörung, schwerer depressiver Störung, Panikattacken, Aufmerksamkeitsdefizitstörung, oder Kombinationen davon.

13. Zusammensetzung zur Verwendung nach Anspruch 12 zur Vorbeugung und/oder Behandlung von Beeinträchtigung der kognitiven Funktionen, Schlaflosigkeit, Angstzuständen, Babyblues oder postpartaler Depression, und Kombinationen davon.

14. Zusammensetzung zur Verwendung nach Anspruch 13, wobei die Beeinträchtigung der kognitiven Funktionen auf arbeitsbedingten Stress zurückzuführen ist.

15. Zusammensetzung zur Verwendung nach Anspruch 14, wobei die beeinträchtigten kognitiven Funktionen das Kurzzeitgedächtnis, Aufmerksamkeit und/oder Entscheidungsfähigkeit sind.

## Revendications

1. Composition comprenant une association probiotique comportant les souches suivantes :
- *Lactobacillus reuteri* PBS072, et
- *Bifidobacterium breve* BB077 ou *Bifidobacterium lactis* BL050 ;
dans laquelle
la souche de *Lactobacillus reuteri* est présente dans la composition en pourcentages en poids par rapport au poids total de l'association probiotique de 20 % à 80 % ;
la souche de *Bifidobacterium breve* est présente dans la composition en un pourcentage en poids par rapport au poids total de l'association probiotique de 10 % à 60 % ;
la souche de *Bifidobacterium lactis* est présente dans la composition en un pourcentage en poids par rapport au poids total de l'association probiotique de 10 % à 60 %.

2. Composition selon la revendication 1, dans laquelle la souche *Lactobacillus reuteri* PBS072 est présente dans la composition en pourcentages en poids par rapport au poids total de l'association probiotique de 30 % à 70 %, de préférence il est égal à 55 %.

3. Composition selon la revendication 1, dans laquelle la souche *Lactobacillus reuteri* PBS072 est présente dans chaque dose unitaire en une quantité allant de 0,5 à 5 milliards d'UFC, de préférence en une quantité allant de 1 à 4 milliards d'UFC, plus préférablement elle est présente en une quantité égale à 2 milliards d'UFC.

4. Composition selon la revendication 1, dans laquelle la souche *Bifidobacterium breve* BB077 est présente dans la composition en un pourcentage en poids par rapport au poids total de l'association probiotique de 20 % à 50 %, de préférence il est égal à 40 %.

5. Composition selon la revendication 1, dans laquelle la souche *Bifidobacterium breve* BB077 est présente dans chaque dose unitaire en une quantité allant de 0,5 à 5 milliards d'UFC, de préférence en une quantité allant de 1 à 4 milliards d'UFC, plus préférablement elle est présente en une quantité égale à 2 milliards d'UFC.

6. Composition selon la revendication 1, dans laquelle la souche *Bifidobacterium lactis* BL050 est présente dans la composition en un pourcentage en poids par rapport au poids total de l'association probiotique de 20 % à 50 %, de préférence il est égal à 40 %.

7. Composition selon la revendication 1, dans laquelle la souche *Bifidobacterium lactis* BL050 est présente dans chaque dose unitaire en une quantité allant de 0,5 à 5 milliards d'UFC, de préférence en une quantité allant de 1 à 2 milliards d'UFC, plus préférablement elle est présente en une quantité égale à 2 milliards d'UFC.

8. Composition selon les revendications 1 à 5, dans laquelle *Lactobacillus reuteri* PBS072*:Bifidobacterium brevis* BB077 sont présents en un rapport pondéral égal à 1:0,7 ou en un rapport de 1:1 s'il est exprimé en UFC.

9. Composition selon les revendications 1 à 3, 6 et 7, dans laquelle *Lactobacillus reuteri* PBS072*:Bifidobacterium lactis* BL050 sont présents en un rapport pondéral de 1:0,7 ou en un rapport de 1:1 s'il est exprimé en UFC.

10. Composition selon les revendications 1 à 9, sous forme orale.

11. Composition selon les revendications 1 à 10, pour une utilisation comme médicament.

12. Composition selon les revendications 1 à 10, pour une utilisation dans la prévention et/ou le traitement d'états pathologiques aigus ou chroniques choisis dans le groupe comprenant l'altération de fonctions cognitives ; l'insomnie ; l'anxiété ; le baby blues ou la dépression post-partum ; la dépression ménopausique ; le trouble anxieux généralisé ; le trouble dépressif majeur ; les crises de panique ; le trouble déficitaire de l'attention ; ou les combinaisons de ceux-ci.

13. Composition pour une utilisation selon la revendication 12, dans la prévention et/ou le traitement de l'altération de fonctions cognitives ; de l'insomnie ; de l'anxiété ; du baby blues ou de la dépression post-partum ; et de leurs combinaisons.

14. Composition pour une utilisation selon la revendication 13, dans laquelle l'altération de fonctions cognitives est due au stress lié au travail.

15. Composition pour une utilisation selon la revendication 14, dans laquelle les fonctions cognitives altérées sont la mémoire à court terme, l'attention et/ou la capacité de prise de décision.
